# DEMANDE DE BREVET EUROPEEN

(11) **EP 1 048 282 A1**
(43) Date de publication de la demande: **02.11.2000**
(21) Numéro de dépôt: 00400863.7
(22) Date de dépôt: 29.03.2000
(51) Int. Cl.: A61K 7/00, A61K 7/48, A61K 7/032, A61K 7/02

(54) **Composition cosmetique comprenant une microdispersion de cire et un polymere colorant**

(30) Priorité: 28.04.1999 FR 9905385
(71) Demandeur: L'OREAL, 75008 Paris (FR)
(72) Inventeur: Piot, Bertrand, 75009 Paris (FR)
(74) Mandataire: Kromer, Christophe

(57) **Abrégé**

L'invention a pour objet une composition cosmétique comprenant une phase aqueuse comprenant une microdispersion de cire et une matière colorante, caractérisée par le fait que la matière colorante comprend au moins un polymère colorant.

La composition peut être utilisée comme composition de maquillage de la peau et des cils.

La composition procure un film de maquillage résistant aux frottements.

## Description

La présente invention a pour objet une composition cosmétique comprenant une microdispersion de cire et un polymère colorant. Plus précisément, l'invention se rapporte à une composition de maquillage de la peau, y compris les lèvres, et des cils, notamment d'êtres humains.

Cette composition peut se présenter sous la forme de mascara, d'eye-liner, de produit pour les lèvres, de fard à joues ou à paupières, de fond de teint, de produit de maquillage du corps, de produit anti-cernes.

Les produits de maquillage de la peau ou des lèvres d'êtres humains comme les eye-liners, les fonds de teints, les rouges à lèvres, ou bien encore des cils comme les mascaras, contiennent généralement des corps gras tels que des cires et des huiles, des pigments et/ou charges et, éventuellement des additifs comme des actifs. Ces compositions sont généralement appliquées sur la peau ou les cils sous forme de couche mince conduisant à la formation d'un film.

Il est aussi connu du document EP-A-557196 des compositions de mascara comprenant une microdispersion aqueuse de cires et un pigment comme matière colorante. Ces mascaras conduisent à un maquillage lisse et régulier.

Le film de maquillage ne présente pas toujours une bonne résistance aux frottements des doigts ou des tissus tels que les vêtements, les serviettes ou les mouchoirs et se désagrège en s'effritant ou en s'étalant. L'effritement du film engendre une perte sensible de l'intensité de la couleur du maquillage, obligeant ainsi la consommatrice à renouveler l'application du produit de maquillage. L'étalement du film forme, quant à lui, une auréole très inesthétique autour de la zone maquillée ; ceci est notamment le cas pour un eye-liner.

D'autre part, pour les peaux à tendance grasse, la sécrétion de sébum favorise le transfert du film de maquillage sur un support venant en contact avec la peau maquillée. Le film n'est donc pas résistant au sébum. Par exemple, pour un eye-liner, lorsque le bord inférieur de la paupière supérieur vient en contact des autres parties de la paupière, le maquillage transfère sur cette partie de peau, provoquant des salissures inesthétiques et une diminution, voire une disparition, du maquillage, ce qui oblige la consommatrice à renouveler l'application du produit d'eye-liner. Ces désagréments sont encore plus accentués lors de frottements avec les doigts ou des tissus (serviettes, mouchoirs), provoquant une destruction importante, voire totale, du film déjà fragilisé.

En outre, ces compositions ont tendances à migrer dans les rides et ridules de la peau, en particulier autour des yeux et des lèvres, entraînant un effet inesthétique.

Le film de maquillage ne présente plus globalement une bonne tenue dans le temps.

La présente invention a donc pour but de proposer une composition ne présentant pas les inconvénients ci-dessus, et conduisant à la formation d'un film ayant une bonne tenue, résistant aux frottements et/ou au sébum, ne migrant pas et ne transférant pas.

La demanderesse a constaté, de façon tout à fait surprenante, que l'utilisation d'un polymère colorant dans une composition de maquillage comprenant une microdispersion aqueuse de cire, pouvait permettre d'obtenir un film de très bonne tenue. Le film obtenu est notamment bien résistant aux frottements, par exemple des doigts ou des tissus, et au sébum. Le film est également souple, flexible, brillant, non collant, ne migre pas et ne transfère pas. En outre, le film présente aussi une bonne résistance à l'eau, lors de baignade ou de douche par exemple, et/ou aux larmes et/ou à la transpiration.

De façon plus précise, la présente invention a pour objet une composition cosmétique comprenant une phase aqueuse comprenant une microdispersion de cire et une matière colorante, caractérisée par le fait que la matière colorante comprend au moins un polymère colorant.

Un autre objet de l'invention est un procédé de maquillage de la peau et/ou des cils et/ou des sourcils consistant à appliquer sur ces derniers une composition telle que définie précédemment.

L'invention a également pour objet l'utilisation dans une composition cosmétique d'au moins un polymère colorant et d'au moins une microdispersion de cire pour obtenir un film de bonne tenue et/ou résistant à l'eau et/ou résistant aux frottements et/ou résistant à la transpiration et/ou résistant au sébum.

Par colorant polymérique, on entend un copolymère à base d'au moins deux monomères distincts dont l'un au moins est un colorant organique monomérique.

De tels colorants polymériques sont connus de l'homme du métier. On peut, par exemple, se référer aux documents : US-5,032,670 ; US-4,999,418 ; US-5,106,942; US-5,030,708 ; US-5,102,980; US-5,043,376 ; US-5,104,913; US-5,281,659, US-5,194,463 ; US-4,804,719 ; WO92/07913.
Il est exposé dans ces documents que ces colorants ont la propriété de ne pas migrer, de ne pas exsuder, de ne pas être extractibles ou sublimables. Ils sont également réputés être stables à la lumière et présenter un fort pouvoir colorant. Toutefois, les milieux dans lesquels leurs mise en oeuvre est envisagée dans ces documents sont des milieux thermoplastiques, essentiellement pour la fabrication d'emballages. Rien ne laisse prévoir les propriétés étonnantes de ces polymères une fois qu'ils sont incorporés dans des compositions cosmétiques.

Les colorants polymériques utilisables dans la présente invention peuvent être de toute nature : polyester, polyamide, polyuréthanne, polyacrylique, poly(méth)acrylique, polycarbonate, leurs mélanges.
Les colorants polymériques utilisables dans la présente invention sont de préférence des polymères polyester ou polyuréthanne. Ces colorants polymériques polyesters ou polyuréthanne peuvent être de type cristallin, semi-cristallin ou amorphe.

Habituellement, les colorants polymériques ont une viscosité intrinsèque d'au moins 0,20 (mesurée selon la méthode décrite dans le brevet US 4804719) et peuvent résulter de la polymérisation de plusieurs monomères dont :
a) lorsqu'il s'agit d'un polymère de la famille des polyesters :
   (i) au moins un résidu acide di-carboxylique ;
   (ii) au moins un résidu diol ; et
   (iii) au moins un monomère colorant.
b) lorsqu'il s'agit d'un polymère de la famille des polyuréthannes :
   (i) au moins un résidu di-isocyanate ;
   (ii) au moins un résidu diol ; et
   (iii) au moins un monomère colorant.

Les résidus acide di-carboxylique peuvent être de type aliphatique, alicyclique ou aromatique, comme par exemple les acides téréphtalique, isophtalique, l'acide sulfophtalique, le sel de sodium de l'acide sulfo-5-phtalique, ou bien encore les acides 1,4-cyclohexane dicarboxylique, 1,3-cyclohexane di-carboxylique, succinique, glutarique, adipique, sébacique, 1, 2-dodécanedioïque, 2,6-naphtalène dicarboxylique, etc.

Dans une variante, les résidus acide dicarboxylique peuvent porter au moins un groupement sulfonique et être choisis parmi les diacides cycloaliphatiques comme le sulfo diacide 1,4-cyclohexane carboxylique, ou bien encore parmi les diacides aromatiques tels que les acides sulfophtaliques, l'acide 4-sulfonaphtalène-2,7-dicarboxylique.

Les résidus di-isocyanate peuvent être de type aliphatique, alicyclique ou aromatique, comme par exemple le 2,4-tolylène di-isocyanate, le 2,6-tolylène di-isocyanate, le 4,4'-biphénylène di-isocyanate, le p-xylène di-isocyanate, le méthylène di-p-phényl di-isocyanate, le p-phénylène di-isocyanate, le m-phénylène di-isocyanate, l'hexaméthylène di-isocyanate, l'isophorone di-isocyanate, etc.

Les résidus diols peuvent être choisi par exemple parmi l'éthylène glycol, le 1,2-propane diol, le 1,3-propane diol, le 2-méthyl-1,3-propanediol, le 1,4-butane diol, le 2,2-diméthyl-1,3-propanediol, le 1,6-hexanediol, le 1,10-décane diol, le 1,12-dodécane diol, le 1,2-cyclohexane diol, le 1,4-cyclohexanediol, le 1,2-cyclohexane diméthanol, le x,8-bis(hydroxyméthyl)-tricyclo-[5.2.1.0]décane, dans lequel x représente 3, 4 ou 5 ; les diols comprenant au moins un atome d'oxygène dans la chaîne comme par exemple le diéthylène glycol, le triéthylène glycol, le dipropylène glycol, le 1,3-bis (2-hydroxyéthyl) benzène, le 1,4-bis (2-hydroxyéthyl)benzène, etc.
En règle générale, ces diols comprennent 2 à 18 et préférentiellement 2 à 12 atomes de carbone.

Les monomères colorants organiques selon l'invention doivent comporter au moins deux substituants susceptibles de réagir avec au moins l'un des autres monomères employés pour la préparation du colorant polymérique et doivent être stables à la température et dans les conditions de préparation dudit polymère.

Hormis ces deux conditions préliminaires, la nature chimique des monomères colorants n'a pas d'importance pour la réalisation de l'invention. Ceux-ci peuvent par exemple, être choisis parmi les anthraquinones, les méthines, bis-méthines, les aza-méthines, les arylidènes, les 3H-dibenzo[7,i-j] isoquinolines, les acides 2,5-diarylaminotéréphtaliques et leurs esters, les phtaloylphénothiazines, les phtaloylphénoxazines, les phtaloylacridone, les anthrapyrimidines, les anthrapyrazoles, les phtalocyanines, les quinophtalones, les indophénols, perinones, les nitroarylamines, benzodifurane, les 2 H-1-benzopyran-2-one, les perylènes, les quinacridones, les triphénodioxazines, les fluoridines, les 4-amino-1,8-naphtalimides, les thioxanthrones, les benzanthrones, les indanthrones, les indigo, thioindigo, xanthène, acridine, azine, oxazine, etc.
On pourra se référer aux brevets US-4,267,306 ; US-4,359,570 ; US-4,403,092 ; US-4,617,373 ; US-4,080,355 ; US-4,740,581 ; US-4,116,923 ; US-4,745,173 ; US-4,804,719, US-5,194,463 ; WO92/07913 pour trouver des exemples de monomères colorants utilisables dans la préparation des colorants polymériques.

Les substituants portés par le monomère colorant et susceptibles de réagir avec les autres monomères peuvent par exemple être choisis parmi les groupements suivants:
- hydroxy,
- carboxy, ester, amino, alkylamino : 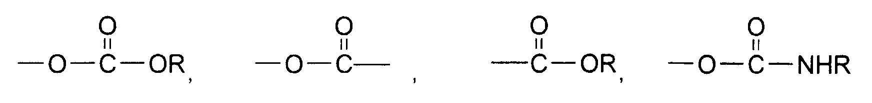
dans lesquels R représente un groupement choisi parmi les alkyles, les aryles. De préférence R est choisi parmi les groupements alkyles en C1-C8 et le phényle. Encore plus préférentiellement R est choisi parmi les groupements méthyle, éthyle et phényle.

Habituellement, le colorant polymérique comprend au moins 5 % en poids de monomère colorant, et n'en comprend pas plus de 55% en poids.
De préférence, le pourcentage en poids de monomère colorant par rapport au poids total du copolymère va de 10 à 40 %.

Pour la préparation des colorants polymériques, on peut se reporter aux procédés décrits dans les documents : US-5,032,670 ; US-4,999,418 ; US-5,106,942 ; US-5,030,708; US-5,102,980 ; US-5,043,376, US-5,194,463; US-4,804,719 ; WO92/07913.

Selon un mode particulier de l'invention, on peut utiliser un polymère colorant sulfopolyester dispersible dans l'eau ayant des groupes de liaison comprenant au moins environ 20 % en moles de carbonyloxy et jusqu'à environ 80 % en moles de carbonylamido, ledit polymère contenant des groupes sulfonate hydrosolubilisants et ayant d'environ 0,01 à environ 40 % en moles, sur la base du total de tous les équivalents d'hydroxy, de carboxy ou d'amino réactifs, de colorant comprenant un ou plusieurs composés organiques thermostables ayant initialement au moins un groupe condensable, que l'on a fait réagir sur ou dans le tronc polymère. Les équivalents susmentionnés englobent leurs divers dérivés condensables, y compris des carbalcoxy, carbaryloxy, N-alkylcarbamyloxy, acyloxy, chlorocarbonyle, carbamyloxy, N-(alkyl)₂ carbamyloxy, alkylamino, N-phénylcarbamyloxy, cyclohexanoyloxy et carbocyclohexyloxy.

Dans une forme préférée de la présente invention, le polymère colorant contient des groupes de liaison carbonyloxy dans la structure moléculaire linéaire, où jusqu'à 80 % desdits groupes de liaison peuvent être des groupes de liaison carbonylamido, le polymère ayant une viscosité inhérente d'environ 0,1 à environ 1,0, mesurée dans une solution à 60-40 parties en poids de phénol/tétrachloroéthane, à 25 °C et à une concentration de 0,25 gramme de polymère dans 100 ml du solvant, le polymère contenant des proportions essentiellement équimolaires d'équivalents d'acide (100 pour cent en moles) par rapport aux équivalents d'hydroxy et d'amino (100 pour cent en moles), le polymère comprenant les résidus de réaction des réactifs (a), (b), (c), (d) et (e) suivants ou de leurs dérivés générateurs d'ester ou générateurs d'estéramide :
(a) au moins un acide dicarboxylique difonctionnel ;
(b) d'environ 4 à environ 25 pour cent en moles, sur la base d'un total de tous les équivalents d'acide, d'hydroxyle et d'amino étant égal à 200 pour cent en moles, d'au moins 1 sulfomonomère difonctionnel contenant au moins un groupe sulfonate cationique relié à un noyau aromatique ou cycloaliphatique où les groupes fonctionnels sont des hydroxy, carboxyle ou amino ;
(c) au moins un réactif difonctionnel choisi parmi un glycol ou un mélange d'un glycol et d'une diamine ayant deux groupes -NRH, le glycol contenant deux groupes -CH₂-OH dont
   (1) au moins 10 pour cent en moles, sur la base du pourcentage total en moles d'équivalents d'hydroxy ou d'hydroxy et d'amino, sont un polyéthylèneglycol de formule structurale :

      H-(OCH₂-CH₂)ₙ-OH

      n étant un entier de 2 à environ 20 ou
   (2) d'environ 0,1 à moins d'environ 15 pour cent en moles, sur la base du pourcentage total en moles d'équivalents d'hydroxy ou d'hydroxy et d'amino, sont un polyéthylèneglycol de formule structurale :

      H-(OCH₂-CH₂)ₙ-OH

      n étant un entier compris entre 2 et environ 500, et à condition que le pourcentage en moles dudit polyéthylèneglycol dans ladite gamme soit inversement proportionnel à la valeur de n dans ladite gamme ;
(d) de zéro à au moins un réactif difonctionnel choisi parmi un acide hydroxycarboxylique ayant un groupe -C(R)₂-OH, un acide aminocarboxylique ayant un groupe -NRH, et un amino-alcool ayant un groupe - C(R)₂-OH et un groupe -NRH, ou des mélanges desdits réactifs difonctionnels ; où chaque R dans les réactifs (c) ou (d) est un atome H ou un groupe alkyle ayant de 1 à 4 atomes de carbone ; et
(e) d'environ 0,1 % en moles à environ 15 % en moles, sur la base d'un total de tous les équivalents d'acide, d'hydroxyle et d'amino étant égal à 200 % en moles, de colorant ayant au moins un groupe acide, hydroxy ou amino que l'on a fait réagir sur ou dans la chaîne polymère.

Avantageusement, le polymère colorant sulfopolyester hydrodispersible comprend :
(a) un monomère acide comprenant de 75 mole % à 84 mole % d'acide isophtalique et de 25 mole % à 16 mole % de sel de sodium de l'acide sulfo-5-isophtalique,
(b) un monomère glycol comprenant de 45 à 60 mole % de diéthylène glycol et de 55 à 40 mole % de 1,4-cyclohexanediméthanol ou d'éthylèneglycol ou leur mélange,
(c) de 0,5 à 10 mole % de monomère colorant
De tels polymères colorant sont décrits dans le brevet US 4804719.

Des colorants polymérique polyuréthane peuvent être par exemple les polymères décrits dans le brevet US-A-5194463 et répondant à la formule (I) suivante : 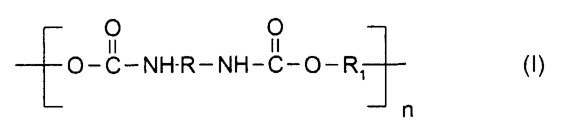 dans laquelle :
R est un radical divalent choisi parmi les radicaux alkylène en C2-C10, cycloalkylène en C3-C8, arylène, alkylène(C1-C4)-arylène-alkylène(C1-C4), alkylène(C1-C4)-cycloalkylène(C3-C8)-alkylène(C1-C4), alkylène(C1-C4)-1,2,3,4, 5,6,7-octahydronaphtalène-2,6-diyl-alkylène(C1-C4),
R¹ est un radical divalent organique comprenant (a) de 1 à 100 moles % de diol organique colorant dans lequel les groupes hydroxyle dudit diol sont liés par un motif alkylène au reste du composé colorant, et (b) de 0 à 99 moles % de diols organiques de formule HO-R²-OH, dans laquelle R² est un radical divalent choisi parmi les radicaux alkylène en C2-C18, cycloalkylène en C3-C8, alkylène(C1-C4)-arylène-alkylène(C1-C4), alkylène(C1-C4)-cycloalkylène(C3-C8)-alkylène (C1-C4), alkylène(C1-C4)-1,2,3,4,5,6,7-octahydronaphtalène-2,6-diyl-alkylène (C1-C4), alkylène(C2-C4)-O-alkylène(C2-C4), alkylène(C2-C4)-S-alkylène(C2-C4), alkylène(C2-C4)-O-alkylène(C2-C4)-O-alkylène (C2-C4),
et n est égal ou supérieur à 2.
De préférence, R1 comprend de 5 à 50 moles pourcent de diol colorant, et n va de 2 à 100.

Pour les polyuréthanes, les monomères diols colorants sont choisis parmi une variété de classes de chromophores. Ces classes de chromophores peuvent être choisis parmi les anthraquinones, les méthines, bis-méthines, les azaméthines, les arylidènes, les 3H-dibenzo[7,i-j] isoquinolines, les phtaloylphénoxazines, les phtaloylacridone, les anthrapyrimidines, les anthrapyrazoles, les phtalocyanines, les quinophtalones, les indophénols, perinones, les nitroarylamines, benzodifurane, les 2 H-1-benzopyran-2-one, les triphénodioxazines, les fluoridines, les benzanthrones, les indigo, thioindigo, xanthène, acridine, azine, oxazine, etc, Ces monomères colorants doivent porter au moins deux groupements hydroxyles.
On pourra se référer aux brevets US-5,194,463 pour trouver des exemples de monomères colorant.

Le colorant polymérique utilisable dans la présente invention peut se présenter sous forme brute, sous la forme d'une poudre dispersible d'une solution en milieu aqueux ou d'une dispersion en milieu aqueux.

Une dispersion en milieu aqueux d'un colorant polymérique insoluble dans l'eau peut être préparée de façon connue à partir d'eau, de colorant polymérique brut tel que décrit dans les documents US-5,032,670 ; US-4,999,418 ; US-5,106,942 ; US-5,030,708; US-5,102,980; US-5,043,376, US-5,194,463. Par exemple on peut partir du colorant polymérique sous forme de poudre ou de granulé, et d'au moins un tensio-actif ionique, de préférence un tensio-actif anionique ou amphotère. Les tensio-actifs anioniques et amphotères peuvent être choisis préférentiellement parmi les sels alcalins d'acides gras en C12-C24, les phosphatides de soja, les phospholipides, les lysophospholipides. Ces tensio-actifs ioniques sont introduits en quantités préférentiellement comprises entre 0,5 et 30%, et encore plus préférentiellement entre 1 et 10% en poids par rapport au poids du colorant polymérique. De façon préférentielle, ces dispersions comprennent en outre au moins un tensioactif non-ionique, que l'on choisit avantageusement parmi les dérivés polyoxyéthylénés ayant un poids moléculaire supérieur à 300, préférablement aux alentours de 1000 à 15000 et une balance hydrophile-lipophile (ou balance HLB) supérieure ou égale à 10.

Par exemple, on peut préparer une dispersion aqueuse de colorant polymérique en suivant les étapes suivantes :
(i) préparation d'une émulsion huile dans eau à partir d'eau, d'une solution du colorant polymérique dans un solvant organique volatile dans lequel il est soluble, en présence d'au moins un tensio-actif ionique et éventuellement d'un tensio-actif non-ionique ;
(ii) évaporation du solvant organique volatile.

Le solvant organique volatile utilisable pour la mise en oeuvre de ce procédé doit être non miscible à l'eau et susceptible de solubiliser le polymère. De préférence son point d'ébullition est inférieur à 100°C. Il peut par exemple être choisi parmi : les solvants hydrocarbonés comme le n-hexane, le cyclohexane, le cyclopentane ; les solvants chlorés comme le chlorure de méthylène, le chloroforme ; les alkyl esters d'acides carboxyliques, comme l'acétate d'éthyle ; les dialkyléthers comme le diisopropyléther. On utilise préférentiellement pour la mise en oeuvre de ce procédé un mélange de solvants comprenant, outre les solvants décrits ci-dessus un solvant volatile, polaire, miscible à l'eau, comme l'acétone ou un alcanol de faible poids moléculaire.

Pour plus d'information sur un tel procédé, on peut se référer aux brevet US 5,043,376 et US 5,104,913.

L'eau des dispersions aqueuses décrites ci-dessus peut ensuite être évaporée, par exemple par atomisation ou par lyophilisation, afin d'obtenir une poudre d'une composition de colorant polymérique dispersible dans tous les milieux.

Le polymère colorant peut être présent dans la composition selon l'invention en une teneur allant de 0,1 % à 40 % en poids de matières sèches, par rapport au poids total de la composition, de préférence de 0,1 % à 35 % en poids et mieux de 0,5 % à 30 % en poids.

La composition selon l'invention comprend par ailleurs une microdispersion aqueuse de cire. On entend par microdispersion de cire, une dispersion aqueuse de particules de cire, dans laquelle la taille desdites particules de cire est inférieure ou égale à environ 1 micron.
Les microdispersions de cire sont des dispersions stables de particules colloïdales de cire, et sont notamment décrites dans "Microemulsions Theory and Practice", L.M. Prince Ed., Academic Press (1977) pages 21-32.
En particulier, ces microdispersions de cire peuvent être obtenues par fusion de la cire en présence d'un tensioactif, et éventuellement d'une partie de l'eau, puis addition progressive d'eau chaude avec agitation. On observe la formation intermédiaire d'une émulsion du type eau-dans-huile, suivie d'une inversion de phase avec obtention finale d'une émulsion du type huile-dans-eau. Au refroidissement, on obtient une microdispersion stable de particules colloïdales solides de cire.

Les particules de la microdispersion de cire ont de préférence des dimensions moyennes inférieures à 1 micron (notamment allant de 0,02 micron à 1 micron), de préférence inférieures à 0,5 micron (notamment allant de 0,05 micron à 0,5 micron).
Ces particules sont constituées essentiellement d'une cire ou d'un mélange de cires. Elles peuvent toutefois comprendre en proportion minoritaire des additifs gras huileux et/ou pâteux, un tensioactif et/ou un additif/actif liposoluble usuel.

Les cires susceptibles d'être utilisées dans les compositions selon l'invention sont choisies parmi les cires, solides et rigides, à température ambiante d'origine animale, végétale, minérale ou de synthèse et leurs mélanges. De préférence, les cires entrant dans la composition peuvent présenter un point de fusion supérieur à 45°C environ, et en particulier supérieur à 55°C, et/ou un indice de pénétration de l'aiguille à 25°C de préférence compris entre 3 et 40, mesuré selon la norme américaine ASTM D 5 ou selon la norme française NFT 004. Les cires sont des substances insolubles dans l'eau et solubles dans les huiles. Elles contribuent à la formation d'un dépôt filmogène, sans pour autant former seules, un film isolable.

On peut notamment citer les cires hydrocarbonées comme la cire d'abeilles, la cire de lanoline, et les cires d'insectes de Chine; la cire de riz, la cire de Carnauba, la cire de Candellila, la cire d'Ouricurry, la cire d'Alfa, la cire de fibres de liège, la cire de canne à sucre, la cire du Japon et la cire de sumac; la cire de montan, les cires microcristallines, les paraffines et l'ozokérite; les cires de polyéthylène, les cires obtenues par la synthèse de Fisher-Tropsch et les copolymères cireux ainsi que leurs esters.
On peut aussi citer les cires obtenues par hydrogénation catalytique d'huiles animales ou végétales ayant des chaînes grasses, linéaires ou ramifiées, en C8-C32. Parmi celles-ci, on peut notamment citer l'huile de jojoba hydrogénée, l'huile de tournesol hydrogénée, l'huile de ricin hydrogénée, l'huile de coprah hydrogénée et l'huile de lanoline hydrogénée.
On peut encore citer les cires de silicone.

Il est également possible d'utiliser des mélanges commerciaux de cires auto-émulsionnables contenant une cire et des tensioactifs. On peut utiliser par exemple la cire commercialisée sous la dénomination 'Cire Auto Lustrante OFR' par Tiscco, qui contient des cires de Carnauba et de paraffine en association avec des tensioactifs non ioniques, ou la cire auto-émulsionnable commercialisée sous la dénomination 'Cerax A.O. 28/B' par La Ceresine, qui contient de la cire d'Alfa en association avec un tensioactif non ionique. Ces mélanges commerciaux permettent de préparer des microdispersions de cires par simple addition d'eau.
On peut encore citer les produits 'Aquacer' de Byk Cera, et notamment : le mélange de cires synthétiques et naturelles avec émulsionnant anionique (Aquacer 520), la cire de polyéthylène avec émulsionnant non ionique ( Aquacer 514 ou 513), la cire polymérique avec émulsionnant anionique (Aquacer 511). On peut également citer le mélange de cires de polyéthylène et de paraffine avec émulsionnant non ionique ' Jonwax 120' de Johnson Polymer.

La composition selon l'invention peut comprendre, de préférence, de 0,1 à 50% en poids de matière sèche de cire, notamment 1 à 30% en poids. Elle peut également comprendre une quantité suffisante de tensioactif pour permettre d'obtenir une microdispersion de cire, ainsi qu'une composition finale, stable. Notamment, elle peut comprendre 0,01 à 30% en poids de tensioactif usuel, pouvant être choisi parmi les composés suivants :
- les tensioactifs anioniques, notamment des sels d'acides gras éventuellement insaturés, ayant par exemple 12 à 18 atomes de carbone; des sels alcalins ou sels de bases organiques des acides alkyl-sulfuriques et alkylsulfoniques ayant 12 à 18 atomes de carbone ou d'acides alkyl-arylsulfoniques dont la chaîne alkyle contient 6-18 atomes de carbone; les éthers-sulfates.
- les tensioactifs non ioniques, notamment des tensioactifs polyalcoxylés et/ou polyglycérolés, et en particulier des acides gras ou amides d'acide gras; des alcools gras ou des alkylphénols; les esters d'acides gras et de polyols; les alcanediols et les alkyléthers d'alcanediols. On peut citer également les alkylcarbamates de triglycérol, les dérivés oxyéthylénés ou propoxylés des alcools de lanoline, des acides gras de la lanoline, ou de leur mélanges.
- les tensioactifs cationiques, notamment les dérivés d'ammonium quaternaire.

La cire ou mélange de cires, peut être associé à un ou plusieurs additifs gras (huileux et/ou pâteux). On peut notamment citer les huiles végétales comme l'huile de tournesol, l'huile de jojoba; les huiles minérales comme l'huile de paraffine; les huiles de silicones; la vaseline, la lanoline; les huiles fluorées; les huiles hydrocarbonées à groupement perfluoré; les esters d'alcools gras.

Il est possible d'introduire en outre dans la phase cireuse microparticulaire des ingrédients actifs liposolubles, tels que des filtres U.V., des vitamines liposolubles, des actifs cosmétiques liposolubles.

Les compositions selon l'invention peuvent se présenter sous différentes formes et en particulier sous forme d'émulsions huile-dans-eau ou eau-dans-huile ou sous forme de dispersions aqueuse.

Selon une forme préférée de réalisation, elles se présentent sous forme d'émulsions huile-dans-eau, qui peut comprendre au moins un tensioactif notamment anionique ou non ionique, en une proportion comprise entre 2 et 30 % en poids par rapport au poids total de la composition.

La composition selon l'invention peut également contenir des ingrédients couramment utilisés en cosmétique, tels que les pigments, les nacres, les charges, les oligo-éléments, les adoucissants, les séquestrants, les parfums, les huiles, les silicones, les épaississants, les vitamines, les protéines, les céramides, les plastifiants, les agents de cohésion ainsi que les agents alcalinisants ou acidifiants habituellement utilisés dans le domaine cosmétique, les émollients, les conservateurs, un polymère filmogène solubilisé ou dispersé dans la phase aqueuse, différent du polymère colorant.

Bien entendu, l'homme du métier veillera à choisir ce ou ces éventuels composés complémentaires, et/ou leur quantité, de manière telles que les propriétés avantageuses de la composition selon l'invention ne soient pas, ou substantiellement pas, altérées par l'adjonction envisagée.

La composition selon l'invention peut être préparée selon les méthodes usuelles des domaines considérés.

La composition de maquillage peut être notamment un mascara, un eye-liner, un produit pour les lèvres (rouge à lèvres), un fard à paupières ou à joues, un produit anti-cernes, un fond de teint, un produit de maquillage du corps du type tatouage provisoire ou semi-permanent.

L'invention est illustrée plus en détail dans les exemples suivants.

### Exemple 1 :

On a préparé une microdispersion de cire de carnauba ayant la composition suivante :
- Cire de carnauba 30 g
- Monostéarate de glycéryle polyoxyéthyléné (30 OE) ( TAGAT S de GOLDSCHMIDT) 7,5 g
- Conservateurs 0,3 g
- Eau qsp 100 g

On a chauffé à 90 °C la cire, le tensioactif et le conservateur en homogénéisant le mélange sous agitation modérée. Puis on a incorporé l'eau chauffée à 90 °C en continuant d'agiter. On a refroidit à température ambiante pour obtenir une microdispersion de cire ayant un diamètre moyen de particules d'environ 285 nm.

On a préparé un mascara ayant la composition suivante :
- Microdispersion de cire de carnauba 69,4 g
- Polymères filmogènes hydrosolubles 2, 75 g
- Gomme arabique 3,3 g
- Polybutylène 0,5 g
- Conservateurs qs
- D-panthénol 5 g
- Colorant sulfopolyester jaune
   (décrit à l'exemple 44 dans EP-A-747036) 1,8 g MA
- Colorant sulfopolyester rouge
   (décrit à l'exemple 10 dans EP-A-747036) 0,7 g MA
- Colorant sulfopolyester bleu
   (décrit à l'exemple 13 dans EP-A-747036) 1,2 g MA
- Soude qs pH = 7
- Eau qsp 100 g

Le mascara s'applique facilement sur les cils et permet d'obtenir un maquillage brillant, présentant une bonne tenue, résistant aux frottements des doigts et au sébum. Le film de maquillage est lisse et homogène.

## Revendications

1. Composition cosmétique comprenant une phase aqueuse comprenant une microdispersion de cire et une matière colorante, caractérisée par le fait que la matière colorante comprend au moins un polymère colorant.

2. Composition selon la revendication 1, caractérisée en ce que le colorant polymérique est choisi parmi les polymères polyester, polyamide, polyuréthanne, polyacrylique, poly(méth)-acrylique, polycarbonate, leurs mélanges.

3. Composition selon l'une quelconque des revendications précédentes, caractérisée en ce que le colorant polymérique est un polyester et résulte de la polymérisation de plusieurs monomères dont :
(i) au moins un résidu acide di-carboxylique ;
(ii) au moins un résidu diol ; et
(iii) au moins un monomère colorant.

4. Composition selon l'une quelconque des revendications 1 à 2, caractérisée en ce que le colorant polymérique est un polyuréthanne et résulte de la polymérisation de plusieurs monomères dont :
(i) au moins un résidu di-isocyanate ;
(ii) au moins un résidu diol ; et
(iii) au moins un monomère colorant.

5. Composition selon l'une des revendications 1 ou 2, caractérisée en ce que le colorant polymérique est un polymère colorant sulfopolyester dispersible dans l'eau ayant des groupes de liaison comprenant au moins environ 20 % en moles de carbonyloxy et jusqu'à environ 80 % en moles de carbonylamido, ledit polymère contenant des groupes sulfonate hydrosolubilisants et ayant d'environ 0,01 à environ 40 % en moles, sur la base du total de tous les équivalents d'hydroxy, de carboxy ou d'amino réactifs, de colorant comprenant un ou plusieurs composés organiques thermostables ayant initialement au moins un groupe condensable, que l'on a fait réagir sur ou dans le tronc polymère.

6. Composition selon l'une quelconque des revendications précédentes, caractérisée en ce que le colorant polymérique comprend 5 % à 55% en poids d'au moins un monomère colorant.

7. Composition selon la revendication 6, caractérisée en ce que le colorant polymérique comprend 10% à 40% en poids d'au moins un monomère colorant.

8. Composition selon l'une quelconque des revendications 3 à 7, caractérisée en ce que le monomère colorant est choisi parmi les anthraquinones, les méthines, bis-méthines, les aza-méthines, les arylidènes, les 3H-dibenzo[7,i-j] isoquinolines, les acides 2,5-diarylaminotéréphtaliques et leurs esters, les phtaloylphénothiazines, les phtaloylphénoxazines, les phtaloylacridone, les anthrapyrimidines, les anthrapyrazoles, les phtalocyanines, les quinophtalones, les indophénols, perinones, les nitroarylamines, benzodifurane, les 2 H-1-benzopyran-2-one, les perylènes, les quinacridones, les triphénodioxazines, les fluoridines, les 4-amino-1,8-naphtalimides, les thioxanthrones, les benzanthrones, les indanthrones, les indigo, thioindigo, xanthène, acridine, azine, oxazine.

9. Composition selon l'une quelconque des revendications précédentes, caractérisée par le fait que le polymère colorant est présent en une teneur en matières sèches allant de 0,1 % à 40% en poids, par rapport au poids total de la composition, de préférence de 0,1 % à 35 % en poids, et mieux de 0,5 % à 30 % en poids.

10. Composition selon l'une quelconque des revendications précédentes, caractérisée par le fait que la microdispersion de cires comprend des particules de cires ayant une taille moyenne inférieure à 1 micron, de préférence inférieure à 0,5 micron.

11. Composition selon l'une quelconque des revendications précédentes, caractérisée par le fait que la cire est choisie dans le groupe formé par les cires ayant un point de fusion supérieur à 45°C environ et/ou un indice de pénétration de l'aiguille à 25°C compris entre 3 et 40.

12. Composition selon l'une des revendications précédentes, caractérisée par le fait que la cire est choisie dans le groupe formé par la cire d'abeilles, la cire de lanoline, les cires d'insectes de Chine; la cire de riz, la cire de Carnauba, la cire de Candellila, la cire d'Ouricurry, la cire d'Alfa, la cire de fibres de liège, la cire de canne à sucre, la cire du Japon et la cire de sumac; la cire de montan, les cires microcristallines, les paraffines et l'ozokérite; les cires de polyéthylène, les cires obtenues par la synthèse de Fisher-Tropsch; les copolymères cireux ainsi que leurs esters; les cires obtenues par hydrogénation catalytique d'huiles animales ou végétales ayant des chaînes grasses, linéaires ou ramifiées, en C8-C32; l'huile de jojoba hydrogénée, l'huile de tournesol hydrogénée, l'huile de ricin hydrogénée, l'huile de coprah hydrogénée, l'huile de lanoline hydrogénée; les cires de silicone; leurs mélanges.

13. Composition selon l'une des revendications précédentes, caractérisé par le fait que la cire est présente en une teneur en matière sèche allant de 0,1 % à 50 % en poids, par rapport au poids total de la composition, et mieux allant de 1 % à 30% en poids.

14. Composition selon l'une des revendications précédentes, dans laquelle les particules de la dispersion de cire comprennent en outre des additifs gras huileux et/ou pâteux et/ou un additif/actif liposoluble usuel.

15. Composition selon l'une des revendications précédentes, comprenant en outre au moins un tensioactif.

16. Composition selon l'une des revendications précédentes, comprenant en outre au moins un polymère filmogène solubilisé ou dispersé dans la phase aqueuse, différent du polymère colorant.

17. Composition selon l'une des revendications précédentes, se présentant sous forme d'émulsion huile-dans-eau ou eau-dans-huile ou sous forme de dispersion aqueuse.

18. Composition selon l'une quelconque des revendications précédentes, caractérisée par le fait qu'elle comprend en outre un additif choisi dans le groupe formé par les pigments, les nacres, les charges, les oligo-éléments, les adoucissants, les séquestrants, les parfums, les huiles, les silicones, les épaississants, les vitamines, les protéines, les céramides, les plastifiants, les agents de cohésion, les agents alcalinisants, les agents acidifiants, les émollients, les conservateurs.

19. Composition selon l'une des revendications précédentes, se présentant sous la forme d'un produit de maquillage tel qu'un produit pour les lèvres, un fond de teint, un fard à joues, un fard à paupières, un eye-liner; un mascara, un produit anti-cernes, un produit de maquillage du corps.

20. Procédé de maquillage de la peau et/ou des cils et/ou des sourcils caractérisé par le fait que l'on applique sur la peau et/ou des cils et/ou des sourcils une composition selon l'une des revendications 1 à 19.

21. Utilisation dans une composition cosmétique d'au moins un polymère colorant et d'au moins une microdispersion de cire pour obtenir un film de bonne tenue et/ou résistant à l'eau et/ou résistant aux frottements et/ou résistant à la transpiration et/ou résistant au sébum.
